# FASCICULE DE BREVET EUROPEEN

(11) **EP 1 924 210 B1**
(45) Date de publication et mention de la délivrance du brevet: **07.10.2009**
(21) Numéro de dépôt: 06808251.0
(22) Date de dépôt: 25.08.2006
(51) Int. Cl.: A61B 17/70

(54) **IMPLANT INTERVERTEBRAL POUR L'ARTICULATION LOMBO-SACREE**
ZWISCHENWIRBELIMPLANTAT FÜR DAS LUMBOSAKRALGELENK
INTERVERTEBRAL IMPLANT FOR THE LUMBOSACRAL JOINT

(30) Priorité: 26.08.2005 FR 0508767
(43) Date de publication de la demande: 28.05.2008
(73) Titulaire: ZIMMER SPINE, 33000 Bordeaux (FR)
(72) Inventeur: PASQUET, Denis, F-33360 Quinsac (FR); LE COUEDIC, Régis, F-78570 Andresy (FR); SENEGAS, Jacques, F-33700 Merignac (FR)
(74) Mandataire: Besnard, Christophe Laurent
(86) Numéro de dépôt international: PCT/FR2006/050811
(87) Numéro de publication internationale: WO 2007/023240

(56) Documents cités:
- EP-A- 1 138 268
- FR-A- 2 858 929
- US-A1- 2004 117 017

## Description

La présente invention a pour objet un implant intervertébral pour l'articulation lombo-sacrée comprenant une cale apte à être disposée entre la cinquième vertèbre lombaire et la vertèbre du sacrum articulée à celle-ci.

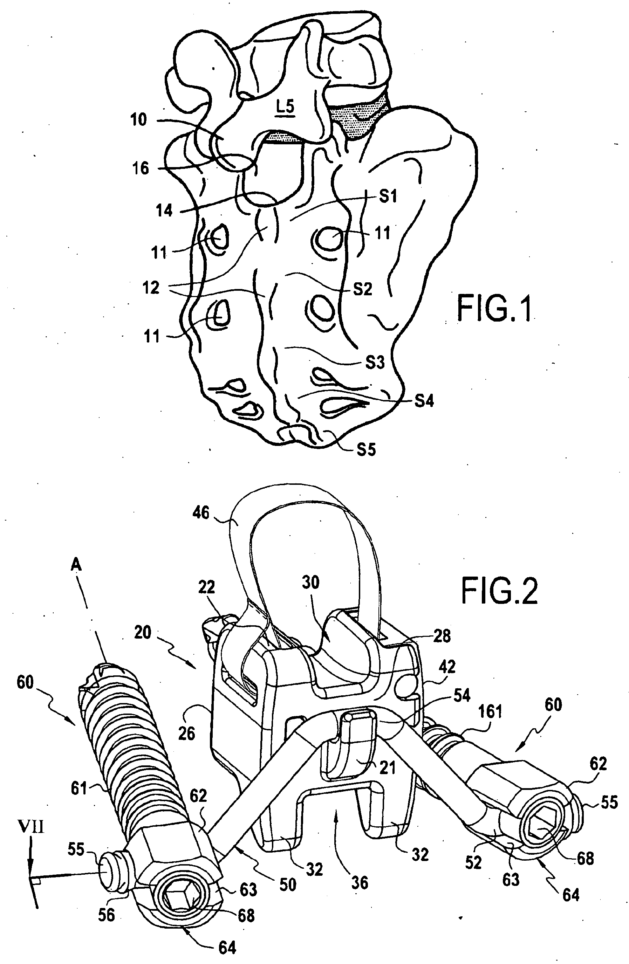

Dans l'anatomie du rachis, comme représenté sur la figure 1, le sacrum est situé en dessous des vertèbres lombaires et est constitué par cinq vertèbres notées S1 à S5 qui, au fil de l'évolution humaine, se sont soudées entre elles. Aujourd'hui, quatre paires des trous, appelés trous de conjugaison 11, subsistent entre les vertèbres sacrées S1 à S5. Ces trous 11, repartis de chaque côté du plan sagittal du rachis, témoignent de l'époque où ces vertèbres étaient séparées.

La vertèbre supérieure du sacrum, notée S1, est articulée à la cinquième vertèbre lombaire, notée L5, comme représenté sur la figure 1. Cette articulation constitue l'articulation lombo-sacrée, ou articulation L5-S1.

Par ailleurs, les vertèbres lombaires présentent une saillie médiane et postérieure : l'apophyse épineuse 10, dénommée ci-après épineuse. Les vertèbres sacrées, elles, ont perdu leurs épineuses au fil de l'évolution, et ne conservent à la place qu'une petite bosse résiduelle 12.

Chez l'homme, certaines douleurs dorsales sont liées aux contraintes s'exerçant sur le disque intervertébral situé les vertèbres L5 et S1, ces contraintes étant, elles mêmes, liées au déplacement des vertèbres L5 et S1 l'une par rapport à l'autre, en particulier lors de l'extension et de la flexion du rachis.

On connaît déjà des implants intervertébraux qui visent à limiter le déplacement des vertèbres L5 et S1 entre elles afin de soulager le disque intervertébral et, notamment, celui décrit dans le document FR 2 858 929.

Cet implant est une cale présentant une face supérieure et une face inférieure opposées, une rainure ménagée sur la face supérieure pour recevoir l'épineuse de la vertèbre L5, et un logement longitudinal, orthogonal à ladite rainure, ménagé sur la face inférieure pour recevoir directement la partie supérieure, ou arc postérieur, de la vertèbre S1.

Cet implant comprend, en outre, deux sangles solidaires de ladite cale, telles que la première sangle est susceptible d'être serrée autour de l'épineuse de la vertèbre L5 pour retenir celle-ci dans ladite rainure, et que la deuxième sangle est passée à travers une ouverture pratiquée dans le sacrum et est susceptible d'être serrée de manière à maintenir la cale contre cette vertèbre S1.

Ce type d'implant engendre deux types de problèmes : sa mise en place oblige à pratiquer une ouverture dans le sacrum et, en fonctionnement, la deuxième sangle frotte contre le sacrum.

La présente invention a pour but de pallier à ces problèmes.

Dans ce but, l'invention a pour objet un implant intervertébral pour l'articulation lombo-sacrée comprenant une cale apte à être disposée entre la cinquième vertèbre lombaire L5 et la vertèbre S1 du sacrum articulée à la vertèbre L5, **caractérisé en ce qu**'il comprend un lien souple dont les portions d'extrémité peuvent être fixées au sacrum à l'aide de moyens de fixation, ce lien souple présentant une partie intermédiaire apte à coopérer avec un système de liaison prévu sur ladite cale de sorte que le lien souple relie la cale au sacrum.

Grâce au lien souple, l'écartement entre la cale et la vertèbre S1 est limité. De préférence, on cherche à éviter tout décollement de la cale par rapport à la vertèbre S1 de façon à limiter au maximum la liberté de mouvement entre les vertèbres L5 et S1 lors de la flexion du rachis. On atteint ce but en jouant sur la longueur et/ou la tension du lien souple lorsque celui-ci est élastique.

Avantageusement, le lien souple est mis sous tension de manière à exercer sur la cale une force de rappel en direction de la vertèbre S1, ce qui permet de plaquer la cale contre cette vertèbre. Selon un premier exemple, pou permettre la mise sous tension du lien souple, les portions d'extrémité de ce lien sont mobiles par rapport auxdits moyens de fixation et peuvent être tirées à l'opposé de ladite cale pour mettre le lien souple sous tension, avant d'être immobilisées par rapport aux moyens de fixation. Dans cet exemple, le lien souple n'est pas nécessairement élastiquement déformable et, avantageusement, il peut coulisser à l'intérieur d'un évidement ménagé dans lesdits moyens de fixation (et être ainsi guidé lorsqu'on le tire par ses extrémités). Selon un deuxième exemple, les portions d'extrémité du lien ne sont pas mobiles par rapport auxdits moyens de fixation et le lien souple est élastiquement déformable, sa longueur étant telle qu'il est sous tension, lorsqu'il coopère avec le système de liaison de la cale et lorsque les moyens de fixation sont en position sur le sacrum.

Selon un premier mode de réalisation de l'invention, lesdits moyens de fixation comprennent des vis pédiculaires qui sont aptes à être ancrées dans le sacrum et qui présentent chacune une tête de vis équipée d'un système de fixation, chaque portion d'extrémité du lien souple étant fixée à ladite tête de vis à l'aide dudit système de fixation.

Le système de fixation retenu doit pouvoir être manipulé facilement par un chirurgien car lors de l'opération l'espace de travail dans la région lombo-sacrée est restreint et la visibilité peut être mauvaise, en particulier à cause du sang.

Avantageusement, ledit système de fixation comprend une partie de corps présentant un évidement susceptible de recevoir une portion d'extrémité dudit lien souple et une vis de serrage pour serrer le lien souple à l'intérieur dudit évidement.

La solution évidement/vis de serrage est de structure simple et facilement manipulable. Par évidement on entend designer tout type d'espace libre approprié. Il peut s'agir d'un trou borgne, d'un trou traversant, d'une rainure... La vis de serrage est mobile par rapport à cet évidement et permet de fixer le lien souple en le serrant à l'intérieur de l'évidement.

Selon un premier exemple d'implant appartenant au premier mode de réalisation précité, la tête de vis des vis pédiculaires forme ladite partie de corps du système de fixation.

Selon un deuxième exemple d'implant appartenant au premier mode de réalisation précité, ladite partie de corps se prolonge par une tige, la tête de vis des vis pédiculaires présentant un évidement susceptible de recevoir ladite tige et une vis de serrage pour fixer cette tige à l'intérieur dudit évidement.

Ce deuxième exemple, de structure plus complexe que le premier, offre la possibilité de déplacer la partie de corps du système de fixation par rapport à la tête de vis. Ceci permet d'orienter chaque partie de corps et son évidement selon une direction préférée et/ou de régler la tension du lien souple après que celui-ci ait été fixé à l'intérieur des évidements des parties de corps et relié à la cale.

Selon un deuxième mode de réalisation de l'invention, lesdits moyens de fixation comprennent des crochets aptes à être accrochés au niveau des trous de conjugaison du sacrum, chaque portion d'extrémité du lien souple étant fixée à ces crochets.

L'invention et ses avantages seront mieux compris à la lecture de la description détaillée qui suit. Cette description fait référence aux figures annexées sur lesquelles :
- la figure 1 représente schématiquement l'anatomie de la région lombo-sacrée du rachis ;
- la figure 2 représente un premier exemple d'implant selon l'invention ;
- la figure 3 représente un deuxième exemple d'implant selon l'invention ;
- la figure 4 est une vue arrière selon la flèche IV, de l'implant de la figure 3, une fois celui-ci mis en place entre les vertèbres L5 et S1 ;
- la figure 5 est une coupe de l'implant de la figure 4 selon le plan médian M et suivant les flèches V ;
- la figure 6 est une coupe axiale d'une tête de vis pédiculaire utilisée dans l'implant de la figure 2 ;
- la figure 7 est une coupe axiale d'une tête de vis pédiculaire utilisée dans l'implant des figures 3 à 5 ;
- la figure 8 est une coupe axiale d'un autre exemple de tête de vis pédiculaire pouvant être utilisé ;
- la figure 9 est une vue arrière représentant un troisième exemple d'implant selon l'invention, mis en place entre les vertèbres L5 et S1 ; et
- la figure 10 est une coupe selon le plan X montrant le lien souple et le crochet de l'implant de la figure 9.

La cinquième vertèbre lombaire L5 et la vertèbre supérieure du sacrum S1 sont représentées schématiquement sur la figure 1. La vertèbre L5 présente dans sa partie médiane et postérieure une épineuse 10. Cette épineuse 10 est située dans le plan sagittal du rachis.

La partie supérieure de la vertèbre S1 forme un arc postérieur 14. La face interne de l'arc postérieur 14 fait face au corps vertébral du sacrum, elle est concave et définit avec le corps vertébral un orifice traversé par la moelle épinière (non représentée), appelé trou rachidien 16.

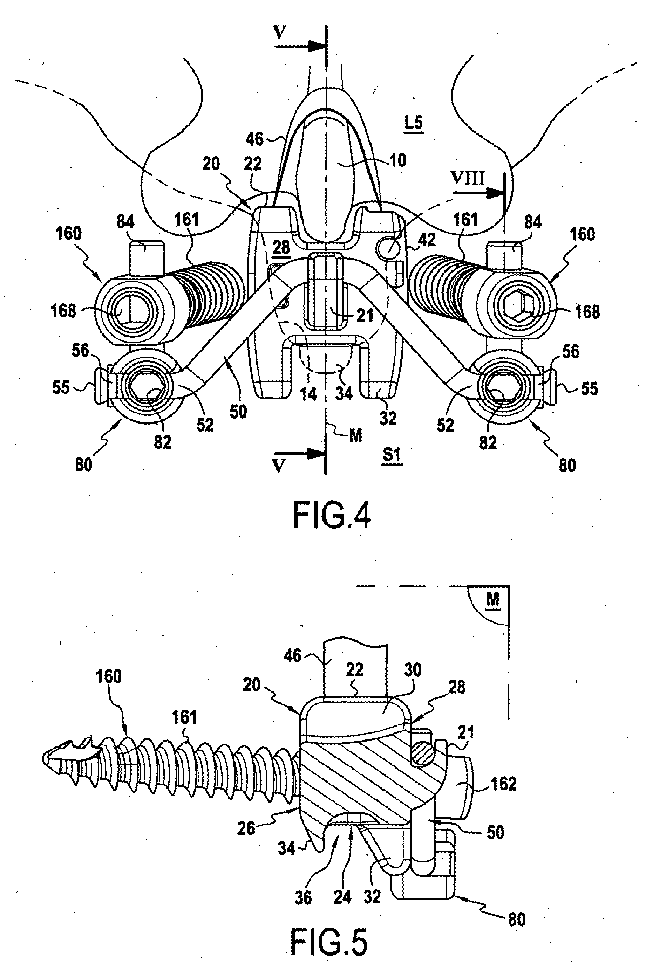

Le même exemple de cale 20 est utilisé pour les deux exemples d'implant représentés sur les figures 2 à 5 et 9.

Cette cale 20 est destinée à être insérée entre l'épineuse 10 et l'arc postérieur 14 et permet de limiter le rapprochement de l'épineuse 10 et de l'arc postérieur 14 lors de l'extension du rachis. Elle est réalisée en matériau biocompatible, par exemple un biopolymère comme le polyétheréthercétone (PEEK).

Les directions haut, bas, avant, arrière, droite et gauche sont définies ci-après par référence au positionnement de la cale sur le rachis, la face avant de la cale étant tournée vers le ventre de l'individu porteur de la cale. Le plan médian M de la cale 20 correspond sensiblement au plan sagittal du rachis lorsque la cale est mise en place, il coupe les faces supérieure 22, inférieure 24, antérieure 26 et postérieure 28 de cette cale.

Une rainure 30, orientée suivant le plan médian M de la cale, est ménagée sur la face supérieure 22 de la cale 20 pour recevoir l'épineuse 10 de la vertèbre L5. La rainure 30 débouche sur les faces antérieure 26 et postérieure 28 de la cale.

Par ailleurs, un logement 36 longitudinal, orienté orthogonalement à ladite rainure 30, est ménagé au niveau de la face inférieure 24 de la cale, pour recevoir la partie supérieure de la vertèbre S1. La cale 20 peut ainsi reposer directement sur la vertèbre S1.

La cale 20 présente à son extrémité inférieure deux extensions 32 dans le prolongement de la face postérieure 28. La cale 20 comprend, en outre, une patte 34 dans le prolongement de la face antérieure 26, qui s'étend en regard de l'espace laissé entre les extensions 32. La patte 34 et les extensions 32 définissent entre elles le logement longitudinal 36 précité. Les extensions 32 sont écartées de manière à pouvoir loger entre elles la bosse résiduelle 12 de la vertèbre S1.

Les exemples d'implant représentés comprennent également des moyens d'attache pour attacher la cale 20 à l'épineuse 10 de la vertèbre L5. Avantageusement, ces moyens d'attache comprennent une sangle 46 solidaire de la cale 20 et susceptible d'être serrée autour de l'épineuse 10 de la vertèbre L5 pour retenir cette épineuse 10 dans la rainure 30 ménagée sur la face supérieure 22 de la cale. Une extrémité de la sangle 46 est fixée à la cale 20, d'un coté de la rainure 30, tandis que l'autre extrémité peut être passée dans un système d'attache 42 solidaire de la cale 20, situé de l'autre côté de rainure 30. Le système d'attache 42 peut être amovible.

Lors de la mise en place de la cale 20, la sangle 46 est passée autour de l'épineuse 10 puis passée dans le système d'attache 42. Ce système 42 peut être autobloquant de sorte qu'une fois la sangle 46 passée dans le système dans une direction et serrée autour de l'épineuse 10, le système 42 empêche la sangle de glisser dans la direction opposée.

La cale 20 comprend en outre un crochet 21 formé sur sa face postérieure 28 et qui sera décrit plus en détail ci-après.

En référence à la figure 2, nous allons maintenant décrire un premier exemple d'implant selon l'invention.

Cet implant comprend une cale 20 du type précité, équipée de moyens d'attache, d'un lien souple 50 et deux vis pédiculaires 60 situées de chaque côté de la cale 20.

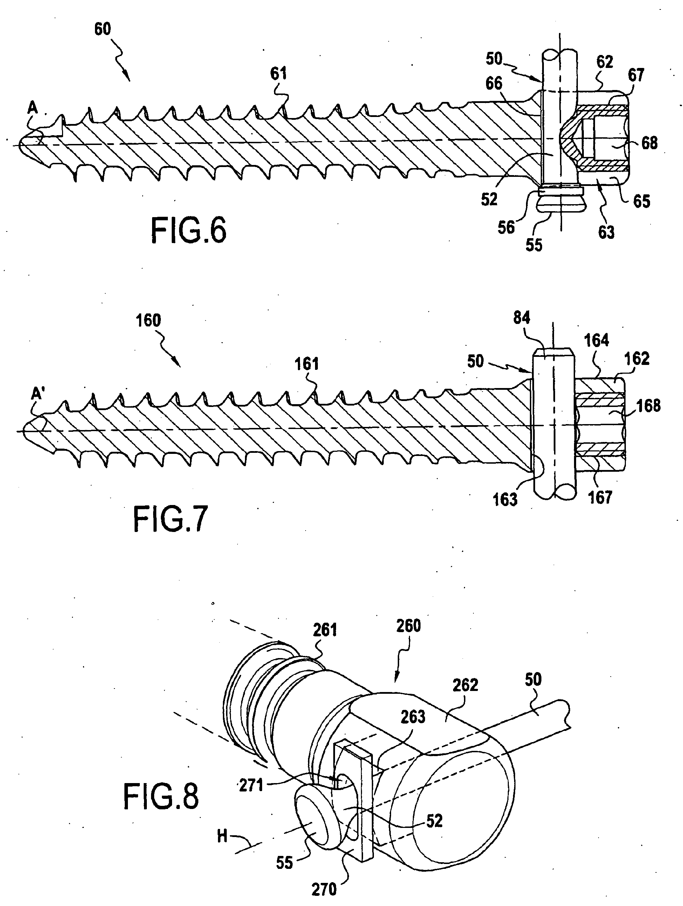

Les vis pédiculaires 60 comprennent un corps de vis 61 apte à être vissé dans le sacrum et une tête de vis 62 destinée à rester, au moins en partie, à l'extérieur du sacrum. Cette tête de vis 62 présente un évidement 63 apte à recevoir une portion d'extrémité 52 du lien souple 50. Dans l'exemple, cet évidement est une rainure ménagée sur la face d'extrémité 64 de la tête de vis. Cette rainure traverse radialement (c'est-à-dire orthogonalement à l'axe A de la vis) la tête de vis 60, d'un bord à l'autre. Cette rainure est délimitée par deux parois latérales opposées 65 et une paroi de fond 66 (voir figure 6). Les parois latérales définissant entre elles, dans la partie médiane de la rainure, un espace libre fileté 67 apte à coopérer avec une vis de serrage 68.

Le lien souple 50 présente, de préférence, une certaine élasticité. Il est par exemple formé d'au moins une tresse tubulaire réalisée à partir de fils de matériaux biocompatibles comme le polyéthylène. Le tressage et, surtout, les propriétés intrinsèques du polyéthylène assurent l'élasticité du lien souple. Les deux portions d'extrémité 52 du lien souple 50 sont destinées à être fixées aux têtes de vis 62. Par ailleurs, une portion intermédiaire 54 du lien souple 20 est apte à coopérer avec un système de liaison présent sur la cale 20. De préférence, cette portion intermédiaire 54 est sensiblement médiane.

Dans l'exemple, le crochet 21 situé à l'arrière de la cale 20 fait office de système de liaison. Ce crochet 21 est réalisé en une seule pièce avec la cale 20 et fait saillie sur la face postérieure 28 de la cale. Il est tourné vers le haut, de manière à retenir la portion intermédiaire 54 du lien souple 50. D'autres systèmes de liaison pourraient cependant être envisagés comme une rainure ménagée à l'arrière de la cale 20, par exemple sur sa face postérieure 28, ou un perçage traversant ladite cale.

La mise en place de l'implant se déroule comme suit. D'abord, on fixe les vis 60 sur le sacrum. Ensuite, on engage les portions d'extrémité 52 du lien souple 50 dans les évidements 63 des têtes de vis 62 et la portion intermédiaire 54 du lien souple 50 dans le crochet 21. Pour s'assurer que les portions d'extrémité 52 du lien souple restent dans les évidements 63 avant d'être fixées définitivement à l'aide de la vis de serrage 68, le lien souple présente à ses extrémités des excroissances 55 de dimensions supérieures à la largeur de l'évidement 63. En outre, des rondelles 56 peuvent être prévues entre les excroissances 55 et les tête de vis 62. Enfin, on fixe chaque portion d'extrémité 52 du lien souple à sa tête de vis 62 respective en serrant la vis de serrage 68. Cette vis vient presser le lien souple contre la paroi de fond 66 de l'évidement 63, pour le maintenir en position (voir figure 6). Pour ne pas endommager le lien souple 50 et risquer de le sectionner, l'extrémité avant de la vis de serrage 68 peut être arrondie.

La tension du lien souple 50 peut être réglée en tirant sur les portions d'extrémité 52 du lien souple pour les éloigner de la cale 20, avant de les fixer définitivement à l'aide des vis de serrage 68 à l'intérieur des évidements 63.

Une fois sous tension, le lien souple 50 exerce sur la cale des forces de rappel ayant une résultante dirigée vers la vertèbre S1. Pour que cette résultante ait une composante verticale dirigée vers le bas, suffisamment importante, il est préférable que les têtes de vis 62 soient situées suffisamment en dessous du crochet 21. En outre, on peut faire en sorte que la portion intermédiaire 54 du lien souple 50 soit située plus en arrière que les portions d'extrémité 52 du lien souple (c'est-à-dire plus en arrière que les têtes de vis 62). Ceci permet à la résultante des forces de rappel d'avoir une composante horizontale dirigée vers le rachis (c'est-à-dire vers l'avant de la cale 20). Une telle composante horizontale empêche la cale 20 de sortir par l'arrière de l'espace intervertébral L5-S1.

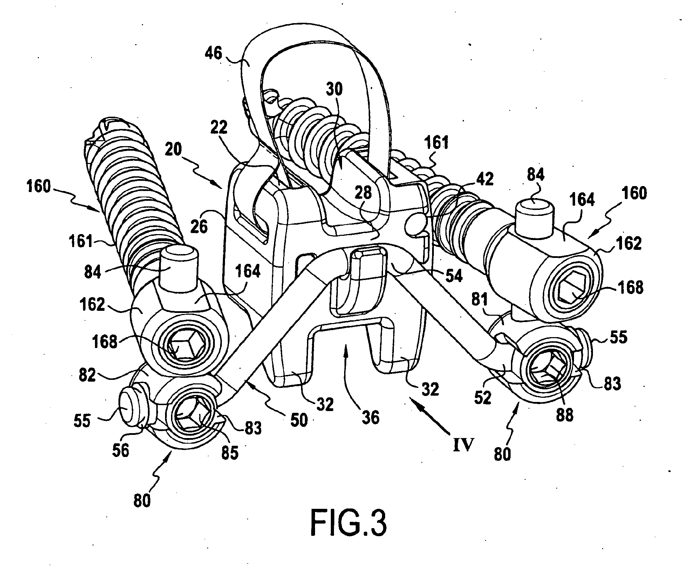

En référence aux figures 3 à 6, nous allons maintenant décrire un deuxième exemple d'implant selon l'invention. La cale 20 et son système d'attache, de même que le lien souple 50 sont identiques à ceux du premier exemple. La différence entre les deux exemples réside dans le système de maintien des portions d'extrémité 52 du lien souple. Le deuxième exemple d'implant représenté comprend, d'une part, deux vis pédiculaires 160 avec un corps de vis 161 et une tête de vis 162 et, d'autre part, deux systèmes de fixation 80 distincts des têtes de vis 162. Chaque système de fixation comprend une partie de corps 82. Cette partie de corps 82 présente un évidement 83 de type rainure, ménagé sur sa face avant et à l'intérieur duquel le lien souple 50 peut être enfoncé. Une vis de serrage 88 est apte à être vissée dans une partie filetée de l'évidement pour serrer le lien souple à l'intérieur de celui-ci et l'immobiliser. En outre, chaque partie de corps 82 est prolongée par une tige 84.

D'autre part, comme représenté sur la figure 7, chaque tête de vis 162 présente un évidement 163, par exemple un perçage qui traverse la tête de vis perpendiculairement à son axe A'. Cet évidement 163 est susceptible de recevoir ladite tige 84. La tige 84 peut alors être fixée à l'intérieur de l'évidement 163 à l'aide d'une vis de serrage 168 coopérant avec un trou fileté 167 ménagé selon l'axe A' et débouchant dans l'évidement 163, de sorte que la vis de serrage 168 est apte à serrer la tige 84 dans l'évidement 163.

Ce deuxième exemple d'implant permet, en faisant coulisser la tige 84 dans l'évidement 163, de régler la tension du lien souple 50. Ainsi, lors de l'installation de l'implant, on peut d'abord fixer la portion intermédiaire 54 du lien souple à la cale 20 et les portions d'extrémité 52 du lien souple au système de fixation 80, en effectuant un premier réglage de la tension du lien souple et, ensuite, fixer les tiges 84 dans leur position définitive pour effectuer, si nécessaire, un deuxième réglage de la tension du lien souple.

En référence à la figure 8, nous allons décrire un autre exemple de moyens de fixation des portions d'extrémité 52 du lien souple 50 au sacrum. Ces moyens de fixation comprennent, d'une part, des vis pédiculaires 260 aptes à être ancrées dans le sacrum et qui présentent chacune un corps de vis fileté 261 et une tête de vis 262 traversée par un trou 263 et, d'autre part, une plaquette 270 percée, enfilée sur le lien souple 50 et retenue à l'extrémité de celui-ci par une excroissance 55 présente sur le lien souple et formée, par exemple, par un noeud ou une boule de fils entrecroisés.

Le contour du trou 263 est tel que la plaquette 270 et le lien souple 50 peuvent traverser le trou 263 lorsque la plaquette 270 est inclinée sensiblement suivant l'axe H du trou, et tel que cette même plaquette 270 ne puisse pas traverser le trou 263 lorsqu'elle est sensiblement perpendiculaire à l'axe H. Par exemple, la plaquette 270 et le contour du trou 263 présentent tous les deux une forme rectangulaire, la largeur de la plaquette étant inférieure à la largeur du contour du trou tandis que la longueur de la plaquette est supérieure à la diagonale du contour du trou. Dans cet exemple, la plaquette 270 doit être inclinée dans le sens de la longueur pour traverser le trou 263.

Pour fixer le lien souple 50 à la tête de vis 262, on passe la plaquette 270, inclinée suivant l'axe H, et la portion d'extrémité 52 du lien souple à travers le trou 263. Une fois le trou 263 traversé, on rabat la plaquette 270 dans une position perpendiculaire à l'axe H. La plaquette 270 a d'ailleurs tendance à revenir dans cette dernière position naturellement. Le lien souple 50 étant sous-tension, la plaquette 270 est plaquée contre la tête de vis 262 dans sa position perpendiculaire à l'axe H de sorte que la portion d'extrémité 52 (retenue par l'excroissance 55 et la plaquette 270 du lien souple 50) ne peut plus s'échapper de la tête de vis 262. Pour faciliter l'inclinaison de la plaquette 270 par rapport au lien souple 50, le perçage 271 à l'intérieur duquel est enfilé le lien souple 50 est de forme oblongue.

Contrairement aux systèmes de fixation représentés sur les figures 6 et 7, celui de la figure 8 ne permet pas de régler la tension du lien souple 50.

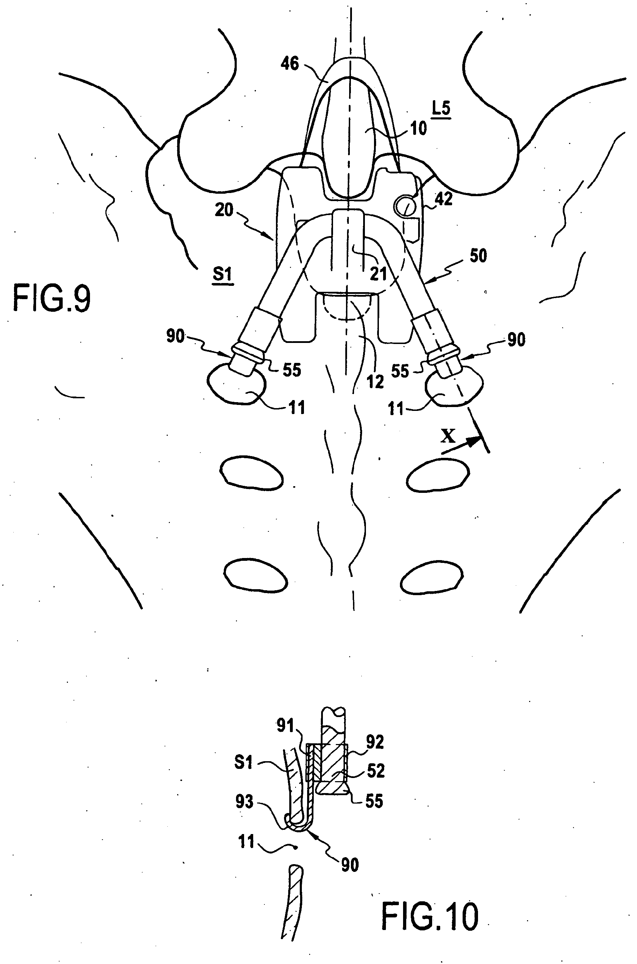

En référence à la figure 9, nous allons maintenant décrire un troisième exemple d'implant selon l'invention. Celui-ci comprend une cale 20 du type de celle précédemment décrite mais ne comprend pas de vis pédiculaire. A la place des vis, cet implant comprend des crochets 90 aptes à être accrochés dans les trous de conjugaison 11 du sacrum, situés sous la cale 20 et de chaque côté de celle-ci.

De préférence, on accroche un crochet 90 dans chacun des deux trous de conjugaison 11 situés entre les vertèbres S1 et S2, c'est-à-dire les trous 11 situés le plus près de la cale 20.

Chaque portion d'extrémité 52 du lien souple 50 est fixée à un crochet par un système de fixation. Dans l'exemple de la figure 10, ce système de fixation comprend un manchon 92 formé sur la partie droite 91 (par opposition à la partie recourbée 93) du crochet 90. La portion d'extrémité 52 du lien souple 50 traverse le manchon 92 et est retenue à l'intérieur de celui-ci par une excroissance 55 formée à l'extrémité du lien souple 50. Cette excroissance a des dimensions supérieures à celles de l'ouverture du manchon 92 de manière à ne pas pouvoir le traverser.

Pour fixer les extrémités 52 du lien souple au crochet 90, d'autres systèmes de fixation pourraient, bien entendu, être envisagés. Ainsi, on pourrait utiliser des systèmes analogues à ceux précédemment décrits et représentés sur les figures 6 à 8, qui équipent les têtes de vis pédiculaires, c'est-à-dire soit un système avec une vis de serrage coopérant avec un évidement, soit un système avec un trou au contour particulier coopérant avec une plaquette.

On notera que quelque soit le mode de réalisation retenu, on obtient une mise sous tension du lien souple 50 de manière à tirer la cale 20 vers le bas.

Dans les modes de réalisation des figures 2 et 3, les portions d'extrémité 52 du lien 50 sont mobiles par rapport auxdits moyens de fixation et peuvent être tirées à l'opposé de ladite cale 20 pour mettre le lien souple sous tension, avant d'être immobilisées par rapport aux moyens de fixation.

Dans l'exemple de la figure 2, le lien souple peut glisser dans les évidements 63 des têtes de vis 62, avant d'être immobilisé par rapport à celles-ci au moyen de la vis de serrage 68.

Dans l'exemple de la figure 3, le lien souple 50 est "doublement" mobile. En effet, d'une part, il peut glisser dans les évidements 83 des parties de corps 82, avant d'être immobilisés par rapport à celles-ci au moyen de la vis de serrage 88 et, d'autre part, les partie de corps 82 peuvent être déplacées avec les portions d'extrémités 52, par rapport aux têtes 162 des vis 160 fixées sur le sacrum.

Dans l'exemple de la figure 9, les portions d'extrémité du lien 50 peuvent être fixes ou mobiles par rapport aux crochets 90. Lorsqu'elles sont fixes, le lien souple 50 est élastiquement déformable. La longueur du lien 50 est choisie telle que, lorsque le lien 50 coopère avec la patte 21 de la cale 20 et lorsque les crochets 90 sont en position sur le sacrum (comme représenté sur la figure 9), le lien 50 soit sous tension.

## Revendications

1. Implant intervertébral pour l'articulation lombo-sacrée comprenant une cale (20) apte à être disposée entre la cinquième vertèbre lombaire L5 et la vertèbre S1 du sacrum articulée à la vertèbre L5, **caractérisé en ce qu'**il comprend un lien souple (50) dont les portions d'extrémité (52) peuvent être fixées au sacrum à l'aide de moyens de fixation, ce lien souple présentant une partie intermédiaire (54) apte à coopérer avec un système de liaison prévu sur ladite cale (20) de sorte que le lien souple relie la cale au sacrum.

2. Implant intervertébral pour l'articulation lombo-sacrée selon la revendication 1, **caractérisé en ce que** lesdits moyens de fixation comprennent des vis pédiculaires (60, 160) qui sont aptes à être ancrées dans le sacrum et qui présentent chacune une tête de vis (62, 162) équipée d'un système de fixation, chaque portion d'extrémité (52) du lien souple étant fixée à ladite tête de vis à l'aide dudit système de fixation.

3. Implant intervertébral pour l'articulation lombo-sacrée selon la revendication 2, **caractérisé en ce que** ledit système de fixation comprend une partie de corps (62, 82) présentant un évidement (63, 83) susceptible de recevoir une portion d'extrémité (52) dudit lien souple et une vis de serrage (68, 88) pour serrer le lien souple à l'intérieur dudit évidement (63, 83).

4. Implant intervertébral pour l'articulation lombo-sacrée selon la revendication 3, **caractérisé en ce que** la tête de vis (62) des vis pédiculaires (60) forme ladite partie de corps du système de fixation.

5. Implant intervertébral pour l'articulation lombo-sacrée selon la revendication 3, **caractérisé en ce que** ladite partie de corps (82) se prolonge par une tige (84), la tête de vis (162) des vis pédiculaires (160) présentant un évidement (163) susceptible de recevoir ladite tige (84) et une vis de serrage (168) pour fixer cette tige (84) à l'intérieur dudit évidement (163).

6. Implant intervertébral pour l'articulation lombo-sacrée selon la revendication 1, **caractérisé en ce que** lesdits moyens de fixation comprennent des crochets (90) aptes à être accrochés au niveau des trous de conjugaison (11) du sacrum, chaque portion d'extrémité (52) du lien souple étant fixée à ces crochets (90).

7. Implant intervertébral pour l'articulation lombo-sacrée selon l'une quelconque des revendications précédentes, **caractérisé en ce que** ledit système de liaison comprend un crochet (21) formé sur ladite cale (20).

8. Implant intervertébral pour l'articulation lombo-sacrée selon l'une quelconque des revendications précédentes, **caractérisé en ce que** les portions d'extrémité du lien souple (50) sont mobiles par rapport auxdits moyens de fixation et peuvent être tirées à l'opposé de ladite cale (20) pour mettre le lien souple sous tension, avant d'être immobilisées par rapport aux moyens de fixation, le lien souple (50) exerçant alors sur la cale (20) une force de rappel en direction de ladite vertèbre S1.

9. Implant intervertébral pour l'articulation lombo-sacrée selon l'une quelconque des revendications précédentes**, caractérisé en ce que** ladite cale (20) présente une face supérieure (22) et une face inférieure (24) opposées, **en ce qu'**une rainure (30), orientée suivant le plan médian (M) de la cale est ménagée sur la face supérieure (22) pour recevoir l'épineuse (10) de ladite vertèbre L5, et **en ce qu'**un logement longitudinal (36), orienté orthogonalement à ladite rainure (30), est ménagé sur la face inférieure (24) pour recevoir la partie supérieure de la vertèbre S1.

10. Implant intervertébral pour l'articulation lombo-sacrée selon l'une quelconque des revendications précédentes, **caractérisé en ce qu'**il comprend, en outre, des moyens d'attache (46) pour attacher la cale (20) à l'épineuse (10) de la vertèbre L5.

## Claims

1. An intervertebral implant for the lumbo-sacral joint, the implant comprising a spacer (20) suitable for being placed between the fifth lumbar vertebra L5 and the vertebra S1 of the sacrum that is articulated to the vertebra L5, the implant being **characterized in that** it includes a flexible tie (50) having end portions (52) that can be fastened to the sacrum with the help of fasteners, the flexible tie presenting an intermediate portion (54) suitable for co-operating with a connection system provided on said spacer (20) in such a manner that the flexible tie connects the spacer to the sacrum.

2. An intervertebral implant for the lumbo-sacral joint, according to claim 1, **characterized in that** said fasteners comprise pedicular screws (60, 160) that are suitable for being anchored in the sacrum, each presenting a screw head (62, 162) fitted with a fastening system, each end portion (52) of the flexible tie being fastened to said screw head with the help of said fastening system.

3. An intervertebral implant for the lumbo-sacral joint, according to claim 2, **characterized in that** said fastening system comprises a body portion (62, 82) presenting a recess (63, 83) suitable for receiving an end portion (52) of said flexible tie and a clamping screw (68, 88) for clamping the flexible tie inside said recess (63, 83).

4. An intervertebral implant for the lumbo-sacral joint, according to claim 3, **characterized in that** the screw head (62) of each pedicular screw (60) forms said body portion of the fastening system.

5. An intervertebral implant for the lumbo-sacral joint, according to claim 3, **characterized in that** said body portion (82) is extended by a rod (84), the screw heads (162) of the pedicular screws (160) each presenting a respective recess (163) suitable for receiving said rod (84) and a clamping screw (168) for fastening said rod (84) inside said recess (163).

6. An intervertebral implant for the lumbo-sacral joint, according to claim 1, **characterized in that** said fasteners comprise hooks (90) suitable for being hooked in the intervertebral foramens of the sacrum, each end portion (52) of the flexible tie being fastened to a said hook (90).

7. An intervertebral implant for the lumbo-sacral joint, according to any preceding claim, **characterized in that** said connection system comprises a hook (21) formed on said spacer (20).

8. An intervertebral implant for the lumbo-sacral joint, according to any preceding claim, **characterized in that** the end portions of the flexible tie (50) are movable relative to said fasteners and can be pulled away from said spacer (20) to put said flexible tie under tension, prior to being prevented from moving relative to the fasteners, the flexible tie (50) then exerting return force on the spacer (20) towards said vertebra S1.

9. An intervertebral implant for the lumbo-sacral joint, according to any preceding claim, **characterized in that** said spacer (20) presents a top face (22) and an opposite bottom face (24), **in that** a groove (30) extending along the midplane (M) of the spacer is formed in the top face (22) to receive the spinous process (10) of said vertebra L5, and **in that** a longitudinal housing (36) extending orthogonally to said groove (30) is formed in the bottom face (24) to receive the top portion of the vertebra S1.

10. An intervertebral implant for the lumbo-sacral joint, according to any preceding claim, **characterized in that** it further comprises an attachment (46) for attaching the spacer (20) to the spinous process (10) of the vertebra L5.

## Patentansprüche

1. Zwischenwirbelimplantat für das Lumbosakralgelenk, mit einem Keil (20), welcher geeignet ist, zwischen dem fünften Lendenwirbel L5 und dem an dem Wirbel L5 angelenkten Wirbel S1 des Kreuzbeins angeordnet zu werden, **dadurch gekennzeichnet, daß** es ein flexibles Verbindungsglied (50) aufweist, dessen Endabschnitte (52) mit Hilfe von Befestigungsmitteln am Kreuzbein befestigt werden können, wobei dieses flexible Verbindungsglied einen Zwischenteil (54) umfaßt, der geeignet ist, mit einem an dem Keil (20) vorgesehenen Verbindungssystem zusammenzuwirken, so daß das flexible Verbindungsglied den Keil mit dem Kreuzbein verbindet.

2. Zwischenwirbelimplantat für das Lumbosakralgelenk, nach Anspruch 1, **dadurch gekennzeichnet, daß** die Befestigungsmittel Pedikelschrauben (60, 160) umfassen, die geeignet sind, im Kreuzbein verankert zu werden, und die jeweils einen Schraubenkopf (62, 162) aufweisen, der mit einem Befestigungssystem ausgestattet ist, wobei jeder Endabschnitt (52) des flexiblen Verbindungsgliedes mit Hilfe des Befestigungssystems an dem Schraubenkopf befestigt ist.

3. Zwischenwirbelimplantat für das Lumbosakralgelenk, nach Anspruch 2, **dadurch gekennzeichnet, daß** das Befestigungssystem einen Körper-Teil (62, 82) umfaßt, der eine Ausnehmung (63, 83), welche geeignet ist, einen Endabschnitt (52) des flexiblen Verbindungsgliedes aufzunehmen, sowie eine Klemmschraube (68, 88) aufweist, um das flexible Verbindungsglied in der Ausnehmung (63, 83) festzuklemmen.

4. Zwischenwirbelimplantat für das Lumbosakralgelenk, nach Anspruch 3, **dadurch gekennzeichnet, daß** der Schraubenkopf (62) der Pedikelschrauben (60) den Körper-Teil des Befestigungssystems bildet.

5. Zwischenwirbelimplantat für das Lumbosakralgelenk, nach Anspruch 3, **dadurch gekennzeichnet, daß** der Körper-Teil (82) durch einen Stift (84) fortgesetzt ist, wobei der Schraubenkopf (162) der Pedikelschrauben (160) eine Ausnehmung (163), die geeignet ist, den Stift (84) aufzunehmen, sowie eine Klemmschraube (168) aufweist, um diesen Stift (84) in der Ausnehmung (163) festzuklemmen.

6. Zwischenwirbelimplantat für das Lumbosakralgelenk, nach Anspruch 1, **dadurch gekennzeichnet, daß** die Befestigungsmittel Haken (90) umfassen, die geeignet sind, im Bereich der Zwischenwirbellöcher (11) des Kreuzbeins eingehakt zu werden, wobei jeder Endabschnitt (52) der flexiblen Verbindungsgliedes an diesen Haken (90) befestigt ist.

7. Zwischenwirbelimplantat für das Lumbosakralgelenk, nach einem der vorhergehenden Ansprüche, **dadurch gekennzeichnet, daß** das Verbindungssystem einen Haken (21) umfaßt, der an dem Keil (20) ausgebildet ist.

8. Zwischenwirbelimplantat für das Lumbosakralgelenk, nach einem der vorhergehenden Ansprüche, **dadurch gekennzeichnet, daß** die Endabschnitte des flexiblen Verbindungsgliedes (50) gegenüber den Befestigungsmitteln beweglich sind und entgegen dem Keil (20) gezogen werden können, um das flexible Verbindungsglied unter Spannung zu setzen, bevor sie gegenüber den Befestigungsmitteln festgelegt werden, wodurch das flexible Verbindungsglied (50) nun auf den Keil (20) eine Rückstellkraft in Richtung des Wirbels S1 ausübt.

9. Zwischenwirbelimplantat für das Lumbosakralgelenk, nach einem der vorhergehenden Ansprüche, **dadurch gekennzeichnet, daß** der Keil (20) eine Oberseite (22) sowie eine Unterseite (24) aufweist, die einander gegenüberliegen, daß eine entlang der Mittelebene (M) des Keils ausgerichtete Nut (30) an der Oberseite (22) ausgebildet ist, um den Dornfortsatz (10) des Wirbels L5 aufzunehmen, und daß eine zu der Nut (30) orthogonal ausgerichtete Längsaufnahme (36) an der Unterseite (24) ausgebildet ist, um den oberen Teil des Wirbels S1 aufzunehmen.

10. Zwischenwirbelimplantat für das Lumbosakralgelenk, nach einem der vorhergehenden Ansprüche, **dadurch gekennzeichnet, daß** es ferner Befestigungsmittel (46) umfaßt, um den Keil (20) am Dornfortsatz (10) des Wirbels L5 zu befestigen.
